# FASCICULE DE BREVET EUROPEEN

(11) **EP 3 174 543 B1**
(45) Date de publication et mention de la délivrance du brevet: **27.11.2019**
(21) Numéro de dépôt: 15775759.2
(22) Date de dépôt: 28.07.2015
(51) Int. Cl.: A61K 31/575, A61K 31/58, A61P 27/16

(54) **DÉRIVÉS DE STÉROLS POUR LE TRAITEMENT DE LA PERTE AUDITIVE NEUROSENSORIELLE ET COMPOSITION CORRESPONDANTE**
STEROL-DERIVATE ZUR BEHANDLUNG VON NEUROSENSORISCHEM HÖRVERLUST UND ENTSPRECHENDE ZUSAMMENSETZUNG
STEROL DERIVATIVES FOR TREATING NEUROSENSORY HEARING LOSS, AND CORRESPONDING COMPOSITION

(30) Priorité: 30.07.2014 FR 1401755
(43) Date de publication de la demande: 07.06.2017
(73) Titulaire: Dendrogenix, 4000 Liège (BE)
(72) Inventeur: DE MEDINA, Philippe, F-31770 Colomiers (FR); PAILLASSE, Michaël, F-31100 Toulouse (FR); ULFENDAHL, Mats, 114 25 Stockholm (SE)
(74) Mandataire: Loyer & Abello
(86) Numéro de dépôt international: PCT/FR2015/000164
(87) Numéro de publication internationale: WO 2016/016518

(56) Documents cités:
- US-A1- 2009 076 160
- DE MEDINA ET AL.: "SYNTHESIS OF NEW ALKYLAMINOOXYSTEROLS WITH POTENT CELL DIFFERENTIATING ACTIVITIES: IDENTIFICATION OF LEADS FOR THE TREATMENT OF CANCER AND NEURODEGENERATIVE DISEASES", JOUR OF MEDICINAL CHEMISTRY, vol. 52, 2009, pages 7765-7777, XP002735709, cité dans la demande
- HAYNES ET AL.: "INTRATYMPANIC DEXAMETHASONE FOR SUDDEN SENSORINEURAL HEARING LOSS AFTER FAILURE OF SYSTEMIC THERAPY", LARYNGOSCOPE, vol. 117, 2007, pages 3-15, XP002735710,
- JORGENSEN ET AL.: "COMETIN IS A NOVEL NEUROTROPHIC FACTOR THAT PROMOTES NEURITE OUTGROWTH AND NEUROBLAST MIGRATION IN VITRO AND SUPPORTS SURVIVAL OF SPIRAL GANGLION NEURONS IN VIVO", EXPERIMENTAL NEUROLOGY, vol. 233, 2012, pages 172-181, XP002735711,

## Description

La présente invention concerne la prise en charge de la perte auditive. Plus précisément, la présente invention concerne l'utilisation d'une molécule de formule I ou un sel pharmaceutiquement acceptable d'un tel composé ; la composition selon l'invention est administrée à un sujet pour éviter sa perte auditive ou restaurer son audition.

La perte auditive est une affection commune, qui touche plus de 360 millions de personnes dans le monde pour l'Organisation Mondiale de la Santé (2012). La perte auditive prise en compte par l'Organisation Mondiale de la Santé, est une perte supérieure à 25 dB. Cette pathologie a d'importantes conséquences néfastes, aussi bien économiques qu'émotionnelles pour les personnes atteintes et pour la société (de Graaf et al., Psychosom Med 64, 61-70), (Fellinger et al., Acta Psychiatr Scand 115,243-5), (Fellinger et al., Soc Psychiatry Psychiatr Epidemiol 40, 737-42), (Mohr et al., Policy Anal Brief H Ser 2, 1-4). De nombreux facteurs, tels que l'âge (50% des humains de plus de 65 ans, 80% des plus de 75 ans sont touchés), les traumatismes sonores, physiques ou émotionnels ou encore des facteurs génétiques peuvent être à l'origine de la dysfonction ou la perte des cellules ciliées puis la dégénérescence du nerf auditif menant à la perte auditive.

On sait que, dans un organe auditif non lésé, le son est transmis à des cellules spécialisées du cerveau par la vibration du tympan, qui transmet l'information mécaniquement à l'oreille interne. Dans l'oreille interne, on trouve des cellules ciliées (ci-après dites HC), qui transforment lesdits signaux mécaniques en signaux électriques, lesquels génèrent une information électrique transmise au cerveau par les neurones d'un ganglion spiral (ci-après dits SGN). Les cellules ciliées sont portées par un organe en colimaçon appelé la « cochlée » ; la cochlée comporte deux chambres spiralées disposées côte à côte et remplies de liquides, périlymphe pour l'une et endolymphe pour l'autre ; entre ces deux chambres sont logées les cellules ciliées.

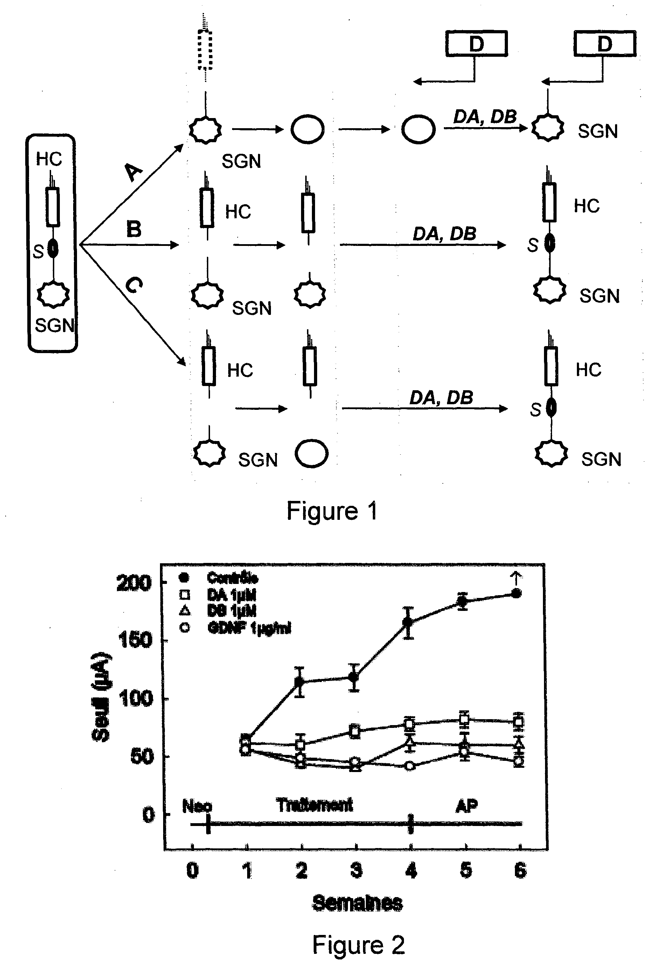

Une perte auditive neurosensorielle moyenne ou sévère peut être due à au moins trois types de disfonctionnements. D'une part, la dysfonction ou la perte partielle des cellules sensorielles HC, d'autre part, la dégénérescence des axones des neurones du ganglion spiral (dites ci-après en abrégé SGN), qui transmettent au cerveau le signal des cellules HC (neuropathie auditive) et enfin, la déstructuration des connexions synaptiques entre les cellules HC et les neurones SGN (synaptopathie auditive). Ces différents mécanismes menant à la perte auditive, sont schématisés sur la figure 1, qui sera détaillée plus loin dans la présente description.

En cas de perte des cellules ciliées, le signal peut être transduit par un implant cochléaire, dont les électrodes remplacent les cellules HC ; chez l'homme, dans un tel cas, les SGN dégénèrent, perdant notamment leurs axones pointant vers les cellules HC, mais ils ne meurent pas. La clé du maintien ou de la récupération de l'audition, réside donc dans la repousse axonale, qui permet de reconstituer les connexions entre les SGN et les transducteurs récepteurs du signal sonore, qu'il s'agisse de cellules HC ou d'un implant cochléaire.

Pour la surdité liée à la perte des cellules HC, les implants cochléaires seuls améliorent l'audition, mais leur efficacité est directement impactée par l'intégrité des SGN, dont ils n'empêchent pas la dégénérescence ; ainsi la recroissance axonale permettant de diminuer la distance entre les terminaisons nerveuses et les électrodes, constitue une approche d'intérêt pour améliorer l'efficacité des implants cochléaires (Shibata et al., Hear Res 281, 56-64). Pour toute autre forme, il n'y a pas, à ce jour, de solution palliative ni curative satisfaisante. Les facteurs neurotrophiques, seules molécules ayant montré un effet bénéfique sur la survie neuronale et la recroissance axonale, ne peuvent pas être utilisés pour cause d'effets secondaires tels que la perte de poids (Winkler et al., Ann Neurol 41, 82-93), une migration cellulaire incontrôlée (Williams, Exp Neurol 113, 31-7) et le risque de cancer lié à la prolifération anormale des cellules de Schwann (Eriksdotter Jonhagen et al., Dement Geriatr Cogn Disord 9, 246-57). L'utilisation de dexaméthasone pour traiter des troubles auditifs a été décrite (Haynes et al., Laryngoscope, 2007, 117, 3-15). Malgré des recherches intensives, il n'y a pas, à ce jour, de solution satisfaite pour résoudre les problèmes susmentionnés et une nouvelle approche est donc nécessaire.

On a déjà décrit des nouveaux dérivés de stérols, composés qui induisent la différentiation neuronale de cellules tumorales embryonnaires pluripotentes, la survie de motoneurones en culture et la prolifération et la différenciation de progéniteurs neuronaux adultes (de Medina et al. 2009, J Med Chem 52, 7765-77) et 2003-------------------)(Khalifa et al., Biochem Biophys Res Commun 446, 681-6). L'homme du métier ne peut évidement pas en déduire que ces composés ont un intérêt pour des pathologies, qui, comme indiqué ci-dessus, sont liés à un problème de connexion neuronale et non à un problème de nombre de neurones dans la zone traitée. Ainsi, l'homme du métier ne chercherait surement pas à utiliser les dérivés de stérols en cause pour reformer les connexions entre d'une part, les neurones SGN et d'autre part, un émetteur de signal constitué par des cellules ciliées au moins partiellement fonctionnelles ou des électrodes d'implants cochléaires.

Or, on a constaté maintenant, selon l'invention, que si l'on utilise ces composés sur un animal de laboratoire rendu sourd par un stress, on obtient un maintien de la transmission de l'information sonore traduite sous forme d'impulsion électrique (par les électrodes d'un implant cochléaire remplaçant les cellules ciliées), l'information étant transmise au travers des SGN vers le cerveau, et ce, sans augmenter le nombre de SGN dudit animal.

La présente invention a, en conséquence, pour objet une composition pharmaceutique pour prévenir une perte d'audition chez un sujet ou pour obtenir une restauration thérapeutique au moins partielle de l'audition d'un sujet traité ayant, avant traitement, une fonction auditive réduite, par mise en contact de ladite composition avec au moins une partie de la cochlée de l'oreille ayant une fonction auditive réduite, ladite composition étant caractérisée en ce qu'elle contient, dans un véhicule pharmaceutiquement acceptable, une dose significativement active d'au moins un composé de formule (I) : 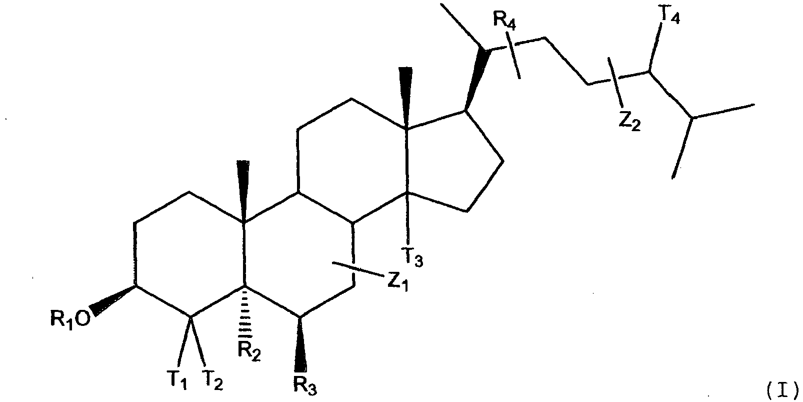 formule dans laquelle :
R₁=H ou R-CO, avec R=H, CH₃ ou C₂H₅ ;
R₂=H ou OH ;
R₃=-NR₅R₆, R₅ étant H ou (CH₂)₃NH₂ et R₆ étant pris dans le groupe formé par -(CH₂)₄NH₂ ; -(CH₂)₃NH (CH₂)₄NH₂ ;
-(CH₂)₄NH (CH₂)₃NH₂ ;
-(CH₂)₃NH(CH₂)₄NH(CH₂)₃NH₂ ; - (CH₂)₃NH₂ ,-(CH₂)₂-imidazol-4-yl et -(CH₂)₂-indol-3-yl;
R₄=H ou OH en position 20,22,24,25,26 ou 27, positionné de façon à obtenir un centre asymétrique de configuration R ou S ;
Z₁ et Z₂ sont les nombres de double liaison (soit 0 soit 1) entre les atomes C7 et C8 ou C22 et C23 respectivement ;
T₁, T₂ et T₃=H ou CH₃, indépendamment les uns des autres ; T₄=H, CH₃, ou C₂H₅, positionné de façon à obtenir un centre asymétrique de configuration R ou S en position 24 et/ou d'au moins un sel pharmaceutiquement acceptable d'au moins un composé de formule (I).

Parmi les compositions ci-dessus définies, on a constaté que les résultats obtenus étaient particulièrement intéressants pour les composés des sous-groupes dans lesquels :
a) le (ou les) composé(s) de formule (I), qu'elle contient, est (sont) défini (s) par Z₁=0 ; R₁=H ; R₂=OH; R₃ = -NHR₆ où R₆ est -(CH₂)₃NH(CH₂)₄NH(CH₂)₃NH₂ ou -(CH₂)₂-imidazol-4-yl; T₁ = T₂ = T₃ = H ;
b) le (ou les) composé(s) de formule (I), qu'elle contient, est (sont) défini (s) par Z₁=0 ou 1, R₁ = H ; R₂ = OH; R₃ = -NHR₆ où R₆ est - (CH₂)₃NH (CH₂)₄NH₂ ou - (CH₂)₄NH(CH₂)₃NH₂ ; T₁ = T₂ = T₃ = H ; R₄ = H ou OH en position 22 ou 27 ;
c) le composé de formule (I), qu'elle contient, est défini par Z₁=0 ; R₁=acétyl ; R₂=OH ; R₄=H ; R₃=NH-(CH₂)₂-imidazol-4-yl et T₁=T₂=T₃=H.

Selon un premier aspect de l'invention, la restauration provoquée par la composition selon l'invention est une amélioration de l'efficacité d'un implant cochléaire préalablement mis en place chez le sujet traité.

Selon un autre aspect de l'invention, la restauration obtenue avec la composition selon l'invention, améliore la fonctionnalité des neurones du ganglion spiral chez le sujet traité, avant que l'on ne pratique, sur ledit sujet, au moins une thérapie destinée à stimuler lesdits neurones ou les cellules ciliées internes et externes.

Selon un autre aspect de l'invention, la restauration provoquée par la composition selon l'invention bénéficie à un sujet, qui requiert la mise en place d'un implant cochléaire en raison d'une perte auditive due à un traumatisme ou à une maladie, ladite restauration maintenant la fonctionnalité des neurones du ganglion spiral avant l'implantation dudit implant cochléaire.

Selon un autre aspect de l'invention, la composition selon l'invention est utilisée pour rendre un sujet plus apte à bénéficier ultérieurement d'une thérapie visant à restaurer tout ou partie de l'oreille interne, ladite thérapie étant choisie dans le groupe formé par la transplantation de cellules souches, la régénération de cellules ciliées par transdifférenciation de cellules supportrices, par la tranfection génique ou par le blocage génique dans toute partie de l'oreille interne.

Selon un autre aspect de l'invention, la composition selon l'invention est administrée par voie orale, intraveineuse, intratympanique, intracochléaire, sur la fenêtre ronde ou ovale de la cochlée intracrâniale, nasale ou sur le tympan.

Selon un autre aspect de l'invention, la composition selon l'invention est mise en place dans l'oreille interne par une électrode imprégnée de ou badigeonnée par ladite composition, ou par une électrode comportant une canule chargée avec ladite composition ou aussi par une électrode composée partiellement d'un ou plusieurs composés de formule (I).

Selon un autre aspect de l'invention, la restauration obtenue par la composition selon l'invention bénéficie à un sujet dont le traumatisme a été généré par un niveau ototoxique de bruit, des agents ototoxiques tels que des radiations, des antibiotiques, des anti-inflammatoires, des agents de chimiothérapie, des métaux lourds ou l'âge du sujet.

Selon un autre aspect de l'invention, la composition selon l'invention permet une restauration qui bénéficie à un sujet dont la perte auditive a été générée par une maladie prise dans le groupe formé par une otite, le syndrome de Pendred, de Niemann-Pick, de Smith-Lemli-Optiz, de Stickler, d'Alport, de Charge, de Jervell et Lange-Nielsen, de Norrie, d'Usher, de Waardenburg et de Perrault, une neurofibromatose de type 2 ou un syndrome bronchiootorénal.

La présente invention a également pour objet l'utilisation d'une composition telle que ci-dessus définie pour maintenir et/ou améliorer la qualité des connexions entre les SGN, d'une part, et les cellules ciliées ou les électrodes d'un implant cochléaire, d'autre part.

La mise en œuvre de l'invention est illustrée par trois exemples auxquels correspond un dessin comportant cinq figures.

La figure 1 est un schéma relatif aux différentes affections de l'oreille interne conduisant aux pertes auditives neurosensorielles auxquelles peut remédier la présente invention, et aux effets cellulaires y relatifs.

Cette figure comporte trois cadres : le cadre de gauche présente la liaison synaptique S d'une cellule HC avec un SGN dans le cas d'un sujet sans affection de l'oreille interne. Le cadre du milieu présente l'état des liaisons SGN/HC dans trois cas de dysfonctionnement A, B, C : dans le disfonctionnement A, le neurone SGN ne reçoit plus d'information de sa synapse S en raison du non fonctionnement de la cellule HC (mise en pointillés) antérieurement reliée à S ; dans le disfonctionnement B, le SGN ne reçoit plus d'information de sa synapse car cette dernière n'assure plus son rôle de liaison (colonne de gauche) et le SGN dégénère (colonne de droite) ; dans le dysfonctionnement C, le SGN n'a plus de synapse réceptrice opérationnelle (colonne de gauche) et l'émission en provenance de la cellule HC ne peut donc alimenter le SGN qui, dès lors, dégénère (colonne de droite). Le cadre de droite présente le résultat quand on fait agir les produits DA ou DB sur les éléments concernés par les dysfonctionnements A (perte de HC), B (synaptopathie) et C (neuropathies) et les représentations de la colonne de droite montrent les états après traitement et récupération partielle de l'audition (le rectangle D schématise une stimulation électrique du SGN).

La figure 2 est un graphe montrant la courbe d'évolution dans le temps de la réponse auditive électriquement évoquée du tronc cérébral (eABR) lorsque le traitement avec la composition selon l'invention démarre deux jours après le début de l'induction de l'ototoxicité par la néomycine.

La figure 3 est un graphe montrant la courbe d'évolution dans le temps de la réponse auditive électriquement évoquée du tronc cérébral (eABR) lorsque le traitement avec la composition démarre seize jours après le début de l'induction de l'ototoxicité par la néomycine.

La figure 4 est un diagramme à barres montrant le nombre de neurones du ganglion spiral dans les expériences relatives aux figures 1 et 2 comparativement à ce que l'on obtient avec une oreille n'ayant pas été exposée à la néomycine (première barre à gauche sur la figure 4).

La figure 5 est une série de trois photographies de lamelles de la cochlée prise au niveau du milieu du modiolus illustrant la quantification du nombre de neurones du ganglion spiral rapporté à la figure 4 ainsi que la recroissance axonale occasionnée par les traitements. Sur chaque photographie, on a indiqué la nature du produit utilisé pour le traitement (AP, DB et GDNF) .

Sur les figures, les abréviations suivantes ont été utilisées :
AP: périlymphe artificielle (acétate de Ringer)
DA: 6β-[2-(1*H*-imidazol-4-yl)-éthylamino]-cholestane-3β,5α-diol
DB: 6β-[3-(4-aminobutylamino)propylamino]-cholestane-3β,5α-diol
eABR : réponse auditive électriquement évoquée du tronc cérébral
Neo: Néomycine
GDNF : facteur neurotrophique issu de la glie
SGC : cellule du ganglion spinal

Le tableau 1 résume l'impact des molécules utilisées dans les exemples 1 à 3 sur la densité des SGN et la réponse auditive électriquement évoquée du tronc cérébral (eABR).

Ci-après, les termes "connexion" et « connexion synaptique" font référence à une interaction fonctionnelle entre les neurones du ganglion spiral et les cellules ciliées ou les électrodes d'un implant cochléaire permettant une stimulation appropriée desdits neurones du ganglion spiral.

Le terme « stress » fait référence à une cause de perte de connexion synaptique fonctionnelle et/ou à la perte des projections des neurones du ganglion spiral.

Les études des exemples 1 à 3 ont été réalisées sur des cochons d'Inde (250-500g). Tous les animaux sont équipés d'une électrode platine-iridium insérée dans la cochlée afin de mimer un implant cochléaire. Les expériences ont été menées selon le protocole décrit par Raphael et ses collaborateurs (Shinohara et al., 2002, Proc. Nati. Acad. Sci. USA, 99, 1657-60).

### Exemple 1 : Effet du traitement précoce avec un dérivé de formule (I) sur l'excitabilité des neurones de ganglion spiral SGN

Les animaux sont anesthésiés (10 mg/kg de xylazine et 40 mg/kg de kétamine en intramusculaire) et l'oreille interne est ouverte par voie post-auriculaire. Une canule pré-remplie contenant 24 µl de sulfate de néomycine 10% a été connectée à une mini-pompe osmotique (ALZET 2002, DURECT Corp., CA, USA) ayant un débit de 0.5 µl/heure. La canule pénètre dans la cochlée à proximité de la fenêtre ronde afin d'atteindre la *scala tympani.* Après 48 heures, la canule a été remplie soit avec une solution de 6β-[2-(1*H*-imidazol-4-yl)-éthylamino]-cholestane-3β,5α-diol (1µM), soit avec une solution de 6β-[3-(4-aminobutylamino)propylamino]-cholestane-3β,5α-diol (1µM), soit avec GDNF (1µg/ml), soit avec de la périlymphe artificielle qui sert de contrôle. Après deux semaines, la pompe est retirée et remplacée par une nouvelle pompe identique pré-remplie de façon similaire. Après deux nouvelles semaines, la pompe est retirée et la canule scellée pour deux semaines supplémentaires. Cette technique est décrite en détail à la page 1658 de la publication Shinohara ci-dessus identifiée.

Les mesures des seuils de réponse auditive électriquement évoquée du tronc cérébral (eABR) sont réalisées à l'aide d'une électrode iridium-platine (Pt-Ir 90%-10%, 250 µm de diamètre) insérée de 1,5mm dans la cochlée (scala tympani) via la fenêtre ronde au moment de la mise en place de la pompe, une électrode de retour (Pt-Ir, 125 µm de diamètre), étant placée contre l'os occipital, sous les muscles de la nuque. La mesure des seuils d'eABR tout au long de l'expérience n'a pas montré de différence significative entre les groupes jusqu'à la deuxième semaine. A partir de là, il y a une diminution significative des seuils d'eABR entre les groupes traités et le groupe contrôle (p< 0,05 à deux semaines et p< 0,001 au-delà de la quatrième semaine), comme montré sur la figure 2. A partir de la sixième semaine, aucune eABR ne peut être obtenue chez les animaux du groupe contrôle. Il n'y a pas de stimulation possible d'eABR sur des animaux placés dans les mêmes conditions que ci-dessus défini, mais non traités par les produits DA ou DB.

### Exemple 2 : Effet d'un traitement retardé réalisé avec les dérivés DA ou DB utilisés dans l'exemple 1 sur l'excitabilité des neurones de ganglion spiral

La procédure utilisée est la même que dans l'exemple 1, à cette différence près que, suite à l'infusion de sulfate de néomycine, les pompes sont remplies de périlymphe artificielle pendant deux semaines. Lors du remplacement, les pompes sont remplacées par des pompes identiques contenant soit une solution de 6β-[2-(1*H-*imidazol-4-yl)-éthylamino]-cholestane-3β,5α-diol (1µM), soit une solution de 6β-[3-(4-aminobutylamino) propylamino]-cholestane-3β,5α-diol (1µM), soit du GDNF (1µg/ml), soit de la périlymphe artificielle, qui sert de contrôle. Ces pompes sont remplacées après deux semaines par des pompes identiques pré-remplies avec les mêmes solutions pour deux semaines supplémentaires.

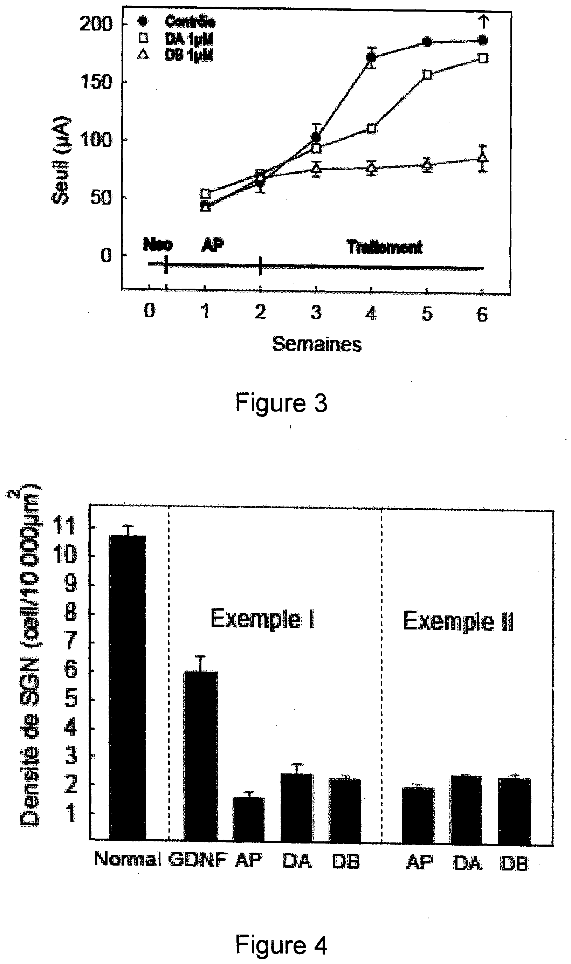

La mesure des seuils de réponse auditive électriquement évoquée du tronc cérébral (eABR) tout au long de l'expérience a montré des différences significatives entre les groupes traités et le groupe contrôle jusqu'à la quatrième semaine. A partir de la cinquième semaine, il n'y a plus de différence significative entre le groupe traité avec 6β-[2-(1*H-*imidazol-4-yl)-éthylamino]-cholestane-3β,5α-diol et le groupe contrôle, alors qu'il y a une différence significative entre le groupe traité avec le 6β-[3-(4-aminobutylamino)propylamino]-cholestane-3β,5α-diol et le groupe contrôle (p< 0,001) comme montré sur la figure 3. Il n'y a pas de stimulation possible d'eABR sur des animaux placés dans les mêmes conditions que ci-dessus défini, mais non traités par les produits DA ou DB.

### Exemple 3 : Quantification de la densité des SGN dans le canal de Rosenthal

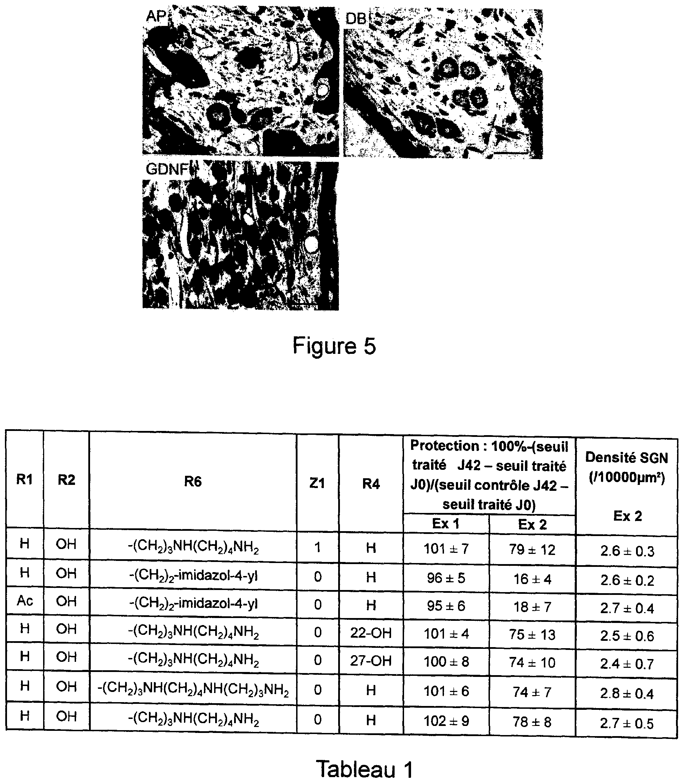

Après la mesure finale du seuil d'eABR, les animaux sont profondément anesthésiés, en intrapéritonal, avec du pentobarbital de sodium (25 mg/kg) et perfusés par voie intracardiaque par une solution saline (37°C), ce qui élimine le sang, puis par une solution de glutaraldéhyde froid (2,5% dans du tampon phosphate 0,1M), ce qui fixe les tissus. L'os temporal est retiré puis la *bulla* ouverte pour dévoiler la cochlée. Une petite fenêtre est ouverte dans l'apex de la cochlée et la membrane de la fenêtre ronde afin de pouvoir laver délicatement la cochlée avec la solution de glutaraldéhyde. La cochlée est ensuite décalcifiée dans une solution d'EDTA (0,1M dans du tampon phosphate) pour permettre d'effectuer des coupes. Après décalcification, la cochlée est déshydratée et incluse dans du « plastic JB-4 » (Polyscience Inc, Warrington, PA). La cochlée est sectionnée en lamelles de 4pm d'épaisseur. Au milieu du *modiolus,* qui est caractérisé par une lamelle, dans laquelle on peut distinguer six sections du canal de Rosenthal, une lamelle sur trois est conservée pour analyse (ce qui évite de compter plusieurs fois les mêmes neurones du ganglion spiral). Les lamelles sont montées sur lames avec du « Paragon », colorées avec du bleu de toluidine et préparées pour la microscopie. Les six sections du canal de Rosenthal de six lamelles sont analysées pour chaque groupe d'animaux (Sigma Pro Scan) pour dénombrer les neurones du ganglion spiral. Les critères retenus pour un SGN sont un diamètre cellulaire entre 14 et 20 µm avec un noyau d'un diamètre de 7 à 10 µm. La densité moyenne de SGN est ainsi calculée et présentée à la figure 4 ; les coupes du canal de Rosenthal sont montrées à la figure 5.

Pour l'exemple 1, seul le traitement avec le GDNF induit une différence significative du nombre de SGN comparativement au groupe contrôle traité avec la périlymphe artificielle (P<0,001). Pour l'exemple 2, on voit qu'aucun traitement retardé n'induit de différence significative comparativement au groupe contrôle.

L'analyse histologique révèle cependant (voir figure 5), pour les animaux traités par DB, que les axones des SGN apparaissent épais et longs alors qu'ils ne sont pas distinguables pour les animaux traités par l'AP. Ceci explique l'efficacité de la DB car la résistance électrique du neurone est d'autant plus faible que la taille de l'axone est grande (réduisant la distance entre le transducteur du signal et le neurone).

## Revendications

1. Composition pour utilisation afin de prévenir une perte d'audition chez un sujet ou afin d' obtenir une restauration au moins partielle de l'audition d'un sujet traité ayant, avant traitement, une fonction auditive réduite, par mise en contact de ladite composition avec au moins une partie de la cochlée de l'oreille ayant une fonction auditive réduite, ladite composition étant **caractérisée en ce qu'**elle contient, dans un véhicule pharmaceutiquement acceptable, au moins un composé de formule (I) : 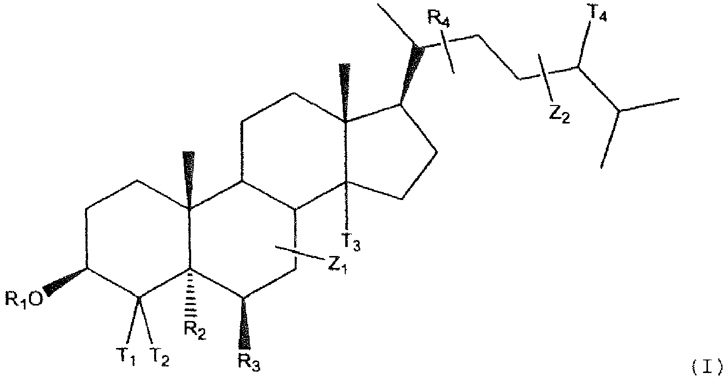 formule dans laquelle R₁ = H ou R-CO avec R = H, CH₃ ou C₂H₅ ; R₂ = H ou OH ; R₃ = -NR₅R₆, R₅ étant H ou - (CH₂)₃ NH₂ et R₆ étant pris dans le groupe formé par -(CH₂)₄ NH₂, - (CH₂)₃ NH (CH₂)₄ NH₂; -(CH₂)₄ NH (CH₂)₃ NH₂; -(CH₂)₃ NH (CH₂)₄ NH(CH₂)₃NH₂ ; - (CH₂)₃ NH₂ ; - (CH₂)₂-imidazol-4-yl et -(CH₂)₂-indol-3-yl; R₄ = H ou OH en position 20, 22, 24, 25, 26 ou 27, positionné de façon à créer un centre asymétrique de configuration R ou S; Z₁ et Z₂ représentent chacun le nombre de double liaison entre les atomes de carbone C7 et C8 et C22 et C23 respectivement (soit 0 soit 1) ; T₁, T₂ et T₃ = H ou CH₃ indépendamment les uns des autres; T₄ = H, CH₃, C₂H₅ positionné de façon à obtenir un centre asymétrique de configuration R ou S en position 24 ; et/ou d'au moins un sel pharmaceutiquement acceptable d'au moins un composé de formule (I).

2. Composition pour utilisation selon la revendication 1, **caractérisée en ce que** le (ou les) composé (s) de formule (I), qu'elle contient, est (sont) défini (s) par Z₁=0, R₁ = H ; R₂ = OH ; R₃ = NHR₆ où R₆ est -(CH₂)₃ NH (CH₂)₄ NH (CH₂)₃ NH₂ ou - (CH₂)₂-imidazol-4yl ; T₁ = T2 = T₃ = H.

3. Composition pour utilisation selon la revendication 1, **caractérisée en ce que** le (ou les) composé(s) de formule (I), qu'elle contient, est (sont) défini(s) par Z₁ = 0 ou 1 ; R₁ = H ; R₂ = OH ; R₃ = -NHR₆ où R₆ est - (CH₂) ₃NH (CH₂)4NH₂ ou - (CH₂)₄ NH (CH₂)₃ NH₂ ; T₁ = T₂ = T₃ = H ; R₄ = H ou OH en position 22 ou 27.

4. Composition pour utilisation selon la revendication 1, **caractérisée en ce que** le composé de formule (I), est défini par Z₁=0 ; R₁=acétyl ; R₂=OH ; R₄=H ; R₃=NH- (CH₂)₂-imidazol-4-yl et T₁=T₂=T₃=H.

5. Composition pour utilisation selon l'une quelconque des revendications 1 à 4, pour améliorer la transmission du signal auditif vers le cerveau depuis le transducteur du signal (cellule ciliée ou électrode).

6. Composition pour utilisation selon la revendication 5, pour améliorer l'efficacité d'un implant cochléaire préalablement mis en place chez un sujet traité.

7. Composition pour utilisation selon la revendication 5, pour améliorer la fonctionnalité des neurones du ganglion spiral chez un sujet traité avant que l'on pratique sur ledit sujet au moins une thérapie destinée à stimuler le nombre et/ou la fonctionnalité desdits neurones ou cellules ciliées internes et externes.

8. Composition pour utilisation selon la revendication 5, pour maintenir la fonctionnalité des neurones du ganglion spiral avant l'implantation d'un implant cochléaire chez un sujet qui requiert la mise en place dudit implant cochléaire en raison d'une perte auditive due à un traumatisme ou une maladie.

9. Composition pour utilisation selon la revendication 5, pour rendre un sujet plus apte à bénéficier ultérieurement d'une thérapie visant à restaurer tout ou partie de son oreille interne, ladite thérapie appartenant au groupe formé par la transplantation de cellules souches, la régénération de cellules ciliées par transdiffférenciation de cellules supportrices, la transfection génique et le blocage génique dans tout ou partie de l'oreille interne.

10. Composition pour utilisation selon l'une des revendications 1 à 4, **caractérisée en ce que** la restauration bénéficie à un sujet, dont le traumatisme a été généré par un niveau ototoxique de bruit, des agents ototoxiques, des radiations, des antibiotiques, des anti-inflammatoires, des agents de chimiothérapie, des métaux lourds, ou le vieillissement du sujet.

11. Composition pour utilisation selon l'une des revendications 1 à 4, **caractérisée en ce que** la restauration bénéficie à un sujet, dont la perte auditive a été générée par une maladie prise dans le groupe formé par une otite, le syndrome de Pendred, de Niemann-Pick, de Smith-Lemli-Opitz, de Stickler, de Charge, de Jervell et Lange-Nielsen, de Norrie, d'Usher, de Waardenburg ou de Perrault, une neurofibromatose de type 2 ou un syndrome bronchio-otorénal.

12. Composition pour utilisation selon l'une quelconque des revendications 1 à 4, pour maintenir et/ou améliorer la qualité des connexions entre les SGN, d'une part, et les cellules ciliées ou les électrodes cochléaires, d'autre part.

## Patentansprüche

1. Zusammensetzung für die Verwendung zur Verhinderung eines Hörverlustes bei einem Patienten oder zur zumindest teilweisen Wiederherstellung des Hörvermögens eines behandelten Patienten, der vor der Behandlung eine verminderte Hörfunktion aufweist, durch in Kontaktbringen der Zusammensetzung mit mindestens einem Teil der Cochlea des Ohrs, das eine verringerte Hörfunktion hat, wobei die Zusammensetzung **dadurch gekennzeichnet ist, dass** sie in einem pharmazeutisch verträglichen Vehikel zumindest eine Verbindung der Formel (I) umfasst: 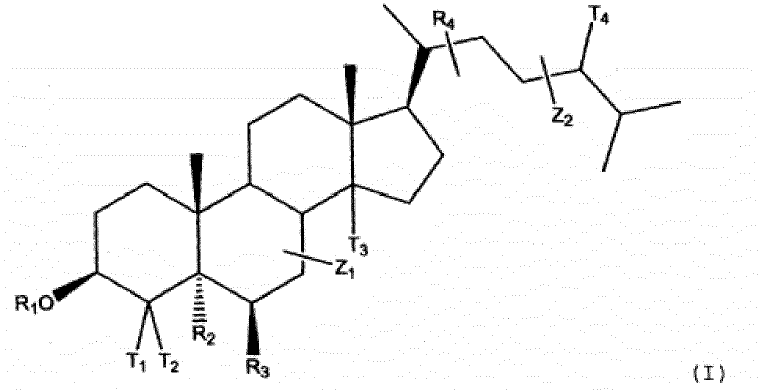 wobei R₁ = H oder R-CO mit R = H, CH₃ oder C₂H₅; R₂ = H oder OH; R₃ = -NR₅R₆, wobei Rs = H oder -(CH₂)₃NH₂, und wobei R₆ aus der Gruppe bestehend aus -(CH₂)₄NH₂, -(CH₂)₃NH(CH₂)₄NH₂; -(CH₂)₄NH(CH₂)₃NH₂; -(CH₂)₃NH(CH₂)₄NH(CH₂)₃NH₂; -(CH₂)₃NH₂; -(CH₂)₂-Imidazol-4-yl und -(CH₂)₂-Indol-3-yl ist; R₄ = H oder OH ist in Position 20, 22, 24, 25, 26 oder 27 positioniert, um ein asymmetrisches Zentrum der R- oder S-Konfiguration zu erzeugen; Z₁ und Z₂ stellen jeweils die Anzahl der Doppelbindungen zwischen den Kohlenstoffatomen C7 und C8 sowie C22 und C23 dar (entweder 0 oder 1); T₁, T₂ und T₃ = H oder CH₃ unabhängig voneinander; T₄ = H, CH₃, C₂H₅ positioniert, um ein asymmetrisches Konfigurationszentrum R oder S in Position 24 zu erhalten; und/oder zumindest ein pharmazeutisch verträgliches Salz von mindestens einer Verbindung der Formel (I).

2. Zusammensetzung für die Verwendung gemäß Anspruch 1, **dadurch gekennzeichnet, dass** die von der Zusammensetzung umfasste (n) Verbindung (-en) der Formel (I) definiert ist (sind) durch Z₁ = 0, R₁ = H; R₂ = OH; R₃ = NHR₆, wobei R₆ ist gleich -(CH₂)₃NH(CH₂)₄NH(CH₂)₃NH₂ oder -(CH₂)₂-Imidazol-4yl; T₁ = T₂ = T₃ = H.

3. Zusammensetzung für die Verwendung gemäß Anspruch 1, **dadurch gekennzeichnet, dass** die von der Zusammensetzung umfasste (n) Verbindung (en) der Formel (I) definiert ist (sind) durch Z₁ = 0 oder 1; R₁ = H; R₂ = OH; R₃ = -NHR₆, wobei R₆ ist gleich -(CH₂)₃NH(CH₂)₄NH₂ oder -(CH₂)₄NH(CH₂)₃NH₂; T₁ = T₂ = T₃ = H; R₄ = H oder OH in Position 22 oder 27.

4. Zusammensetzung für die Verwendung gemäß Anspruch 1, **dadurch gekennzeichnet, dass** die Verbindung der Formel (I) durch Z₁ = 0; R₁ = Acetyl; R₂ = OH; R₄ = H; R₃ = NH-(CH₂)₂-Imidazol-4-yl und T₁ = T₂ = T₃ = H definiert ist.

5. Zusammensetzung für die Verwendung gemäß einem der Ansprüche 1 bis 4 zur Verbesserung der Übertragung des Hörsignals vom Signalwandler (Flimmerzelle oder Elektrode) zum Gehirn.

6. Zusammensetzung für die Verwendung gemäß Anspruch 5 zur Verbesserung der Wirksamkeit eines Cochleaimplantats, das zuvor bei einem behandelten Patienten eingesetzt wurde.

7. Zusammensetzung für die Verwendung gemäß Anspruch 5 zur Verbesserung der Funktionalität von Neuronen des Spiralganglions bei einem behandelten Patienten, bevor an diesem mindestens eine Therapie zur Stimulierung der Anzahl und / oder der Funktionalität der internen und externen Neuronen oder Haarzellen durchgeführt wird.

8. Zusammensetzung für die Verwendung gemäß Anspruch 5 zur Erhaltung der Funktionalität von Neuronen des Spiralganglions vor der Implantation eines Cochleaimplantats bei einem Patienten, der die Einsetzung des Cochleaimplantats aufgrund eines Hörverlusts durch ein Trauma oder eine Krankheit benötigt.

9. Zusammensetzung für die Verwendung gemäß Anspruch 5, um es einem Patienten zu ermöglichen, von einer nachfolgenden Therapie zur Wiederherstellung seines gesamten Innenohrs oder eines Teils davon besser zu profitieren wobei die Therapie aus der Gruppe bestehend aus Stammzelltransplantation, die Haarzellenregenerierung, durch Transdifferenzierung der unterstützenden Zellen, genetische Transfektion und genetische Blockade im gesamten oder einem Teil des Innenohrs, ausgewählt ist.

10. Zusammensetzung für die Verwendung gemäß einem der Ansprüche 1 bis 4, **dadurch gekennzeichnet, dass** die Wiederherstellung einem Patienten zugute kommt, dessen Trauma durch einen ototoxischen Geräuschpegel, ototoxische Mittel, Strahlungen, Antibiotika, entzündungshemmende Mittel, chemotherapeutische Mittel, Schwermetalle oder Alterung des Patienten verursacht wurde.

11. Zusammensetzung für die Verwendung gemäß einem der Ansprüche 1 bis 4, **dadurch gekennzeichnet, dass** die Wiederherstellung einem Patienten zugute kommt, dessen Hörverlust durch eine Krankheit aus der Gruppe bestehend aus Otitis, Pendred Syndrom, Niemann-Pick Syndrom, Smith-Lemli-Opitz Syndrom, Stickler Syndrom, Charge Syndrom, Jervell Syndrom und Lange-Nielsen Syndrom, Norrie Syndrom, Usher Syndrom, Waardenburg Syndrom oder Perrault Syndrom, Neurofibromatose Typ 2 oder bronchio-otorenales Syndrom verursacht wurde.

12. Zusammensetzung für die Verwendung gemäß einem der Ansprüche 1 bis 4 zur Erhaltung und/oder Verbesserung der Qualität der Verbindungen zwischen einerseits den Neuronen des Spiralganglions und andererseits den Haarzellen oder den Cochlea-Elektroden.

## Claims

1. A composition for use intended to prevent hearing loss in a subject or to obtain at least partial restoration of the hearing in a treated subject having reduced auditory function prior to treatment, by placing said composition in contact with at least part of the cochlea of the ear having a reduced auditory function, said composition being **characterised in that** it contains, in a pharmaceutically acceptable vehicle, at least one compound of formula (I): 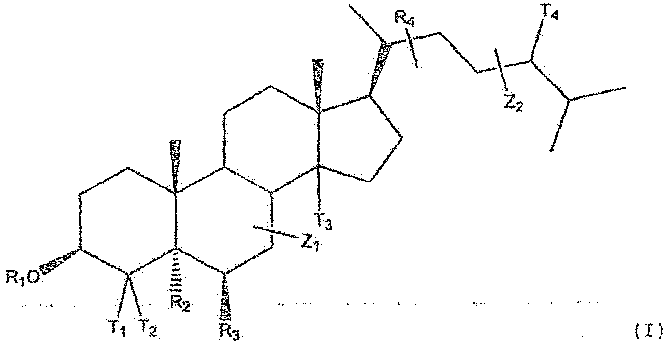 in which formula R₁=H or R-CO, with R=H, CH₃ or C₂H₅; R₂=H or OH; R₃=-NR₅R₆, R₅ being H or -(CH₂)₃NH₂ and R₆ being taken from the group formed by -(CH₂)₄NH₂; - (CH₂)₃NH (CH₂)₄NH₂; -(CH₂)₄NH (CH₂)₃NH₂ ; - (CH₂)₃NH (CH₂)₄NH (CH₂)₃NH₂; -(CH₂)₃NH₂; -(CH₂)₂-imidazol-4-yl and -(CH₂)₂-indol-3-yl; R₄=H or OH in position 20, 22, 24, 25, 26 or 27, positioned so as to obtain an asymmetric centre of configuration R or S; Z₁ and Z₂ each represent the number of double bonds between the atoms C7 and C8 and C22 and C23 respectively (either 0 or 1); T₁, T₂ and T₃=H or CH₃, independently of each other; T₄=H, CH₃, or C₂H₅, positioned so as to obtain an asymmetric centre of configuration R or S in position 24; and/or at least one pharmaceutically acceptable salt of at least one compound of formula (I).

2. A composition for use according to claim 1, **characterised in that** the compound(s) of formula (I), contained therein, is (are) defined by Z₁=0; R₁=H; R₂=OH; R₃ = NHR₆ where R₆ is - (CH₂)₃NH (CH₂)₄NH (CH₂)₃NH₂ or -(CH₂)₂-imidazol-4-yl; T₁ = T₂ = T₃ = H.

3. A composition for use according to claim 1, **characterised in that** the compound(s) of formula (I), contained therein, is (are) defined by Z₁=0 or 1; R₁ = H; R₂ = OH;
R₃ = -NHR₆ where R₆ is - (CH₂)₃NH (CH₂)₄NH₂ or
-(CH₂)₄NH(CH₂)₃NH₂; T₁ = T₂ = T₃ = H; R₄ = H or OH in position 22 or 27.

4. A composition for use according to claim 1, **characterised in that** the compound of formula (I) is defined by Z₁=0; R₁=acetyl; R₂=OH; R₄=H; R₃=NH- (CH₂)₂-imidazol-4-yl and T₁=T₂=T₃=H.

5. A composition for use according to any one of claims 1 to 4 for improving the transmission of the auditory signal towards the brain from the transducer of the signal (hair cell or electrode).

6. A composition for use according to claim 5 for improving the efficacy of a cochlear implant previously positioned in a treated subject.

7. A composition for use according to claim 5 for improving the functionality of the spiral ganglion neurons in a treated subject before at least one therapy intended to stimulate the number and/or the functionality of said neurons or internal and external hair cells has been performed on said subject.

8. A composition for use according to claim 5 for maintaining the functionality of the spiral ganglion neurons prior to implantation of a cochlear implant in a subject requiring the placement of said cochlear implant due to a hearing loss caused by a traumatism or a disease.

9. A composition for use according to claim 5 for making a subject more able to benefit later from a therapy aimed at restoring all or part of the inner ear, said therapy being selected from the group formed by transplantation of stem cells, regeneration of hair cells by transdifferentiation of supporting cells, gene transfection, and gene blocking in all or part of the inner ear.

10. A composition for use according to any one of claims 1 to 4, **characterised in that** the restoration benefits a subject whose traumatism was generated by an ototoxic level of noise, ototoxic agents, radiations, antibiotics, anti-inflammatories, chemotherapy agents, heavy metals, or the ageing of the subject.

11. A composition for use according to any one of claims 1 to 4, **characterised in that** the restoration benefits a subject whose hearing loss was generated by a disease taken from the group formed by otitis, Pendred syndrome, Niemann-Pick disease, Smith-Lemli-Optiz syndrome, Stickler syndrome, Alport syndrome, CHARGE syndrome, Jervell and Lange-Nielsen syndrome, Norrie disease, Usher syndrome, Waardenburg syndrome or Perrault syndrome, a neurofibromatosis type 2, or a branchio-oto-renal syndrome.

12. A composition for use according to any one of claims 1 to 4 for maintaining and/or improving the quality of the connections between the SGNs on the one hand and the hair cells or the cochlear electrodes on the other hand.
